(19) Europäisches Patentamt
European Patent Office
Office européen des brevets

(11) Veröffentlichungsnummer : **0 051 143**
**B1**

# (12) EUROPÄISCHE PATENTSCHRIFT

(45) Veröffentlichungstag der Patentschrift : **04.07.84**

(51) Int. Cl.³ : **C 12 P 33/06**, C 07 J 53/00// C07J71/00

(21) Anmeldenummer : **81107458.2**

(22) Anmeldetag : **19.09.81**

## (54) Verfahren zur Herstellung von 3-beta,7-beta-Dihydroxy-delta-5-steroiden.

(30) Priorität : **03.11.80 DE 3042136**

(43) Veröffentlichungstag der Anmeldung : **12.05.82 Patentblatt 82/19**

(45) Bekanntmachung des Hinweises auf die Patenterteilung : **04.07.84 Patentblatt 84/27**

(84) Benannte Vertragsstaaten : **AT BE CH DE FR GB IT LI LU NL SE**

(56) Entgegenhaltungen :
**EP-A- 0 014 991**
**US-A- 3 429 778**
**CHEMICAL ABSTRACTS, Band 75, Nr. 11, 13. September 1971, Seite 142, Nr. 72841w, Columbus, Ohio, U.S.A. H.J. BRODIE et al.: "Microbiological hydroxylation of estr-4-ene-3, 17-dione"**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Petzoldt, Karl, Dr.**
**Flachsweg 10**
**D-1000 Berlin 38 (DE)**
Erfinder : **Wiechert, Rudolf, Prof.**
**Petzower Strasse 8 A**
**D-1000 Berlin 39 (DE)**
Erfinder : **Laurent, Henry, Dr.**
**Glambecker Weg 21**
**D-1000 Berlin 28 (DE)**
Erfinder : **Nickisch, Klaus, Dr.**
**Alt Lichtenrade 11**
**D-1000 Berlin 49 (DE)**
Erfinder : **Bittler, Dieter**
**Bölkauer Pfad**
**D-1000 Berlin 27 (DE)**

**EP 0 051 143 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft das in den Ansprüchen gekennzeichnete Verfahren.

3β,7β-Dihydroxy-$\Delta^5$-steroide der allgemeinen Formel I gemäß Anspruch 1 sind wichtige Zwischenprodukte zur Partialsynthese pharmakologisch wirksamer Steroide.

Es ist mit Hilfe des erfindungsgemäßen Verfahrens möglich, diese Verbindungen in hoher Ausbeute aus den entsprechenden in 7-Position unsubstituierten Verbindungen darzustellen.

Der erste Reaktionsschritt des erfindungsgemäßen Verfahrens wird unter den Bedingungen durchgeführt, die man üblicherweise bei der mikrobiologischen Hydroxylierung von Steroiden mit Pilzkulturen anwendet. So werden zunächst in allgemein üblichen Vorversuchen die günstigsten Fermentationsbedingungen, wie zum Beispiel Auswahl des günstigsten Nährmediums, des geeigneten Substratlösungs- oder Suspensionsmittels, der Substratkonzentration, der technischen Bedingungen wie Temperatur, Belüftung, pH-Wert und der optimalen Zeiten für Germination, Substratzugabe und Substratkontakt am Enzym des Mikroorganismus analytisch, insbesondere dünnschichtchromatographisch, ermittelt. Dabei hat sich gezeigt, daß es zweckmäßig ist, Konzentrationen von etwa 100-2 000 mg Substrat pro Liter Nährmedium einzusetzen. Der pH-Wert wird vorzugsweise auf einen Wert im Bereich von 5-7 eingestellt. Die Züchtungstemperatur liegt im Bereich von 20-40 °C, vorzugsweise von 25-35 °C. Zur Belüftung werden vorzugsweise 0,5 bis 5 Liter Luft pro Minute pro Liter Kulturbrühe zugeführt. Die Umwandlung des Substrates wird zweckmäßigerweise durch dünnschichtchromatographische Analyse von Probeextrakten verfolgt. Die Fermentationsdauer beträgt etwa 20 bis 80 Stunden.

Nach erfolgter Fermentation werden die Fermentationsprodukte in an sich bekannter Weise isoliert. Die Isolierung kann zum Beispiel in der Weise erfolgen, daß man die Fermentationsansätze mit einem nicht mit Wasser mischbaren organischen Lösungsmittel wie Äthylacetat, Butylacetat oder Methylisobutylketon, extrahiert, die Extrakte einengt und die so erhaltenen Rohprodukte gegebenenfalls durch Chromatographie und/oder Kristallisation reinigt.

Bevorzugte Ausgangsverbindungen des erfindungsgemäßen Verfahrens sind solche der allgemeinen Formel II, welche als Sustituenten R ein Wasserstoffatom, eine Acetylgruppe, eine Propionylgruppe oder Butyrylgruppe tragen.

Es ist überraschend, daß die $\Delta^5$-Steroide der allgemeinen Formel II in der 7β-Position hydroxyliert werden, denn es ist bekannt, daß die entsprechenden $\Delta^4$-Steroide mit Bortryodiplodia malorum in der 7α-Position hydroxyliert werden (Europäische Patentanmeldung 0 014 991).

Es ist seit langem bekannt, daß der selektive Schutz der 3-Hydroxygruppe von 3β,7β-Dihydroxy-$\Delta^5$-steroiden erhebliche Schwierigkeiten bereitet. (J. Amer. Chem. Soc., *74,* 1952, 3310). Die Umsetzung der Verbindungen mit beispielsweise Trimethylacetylchlorid oder Triphenylchlormethan-Agentien, die man konventionellerweise zum selektiven Schutz von sterisch ungehinderten Hydroxygruppen neben sterisch gehinderten Hydroxygruppen anwendet — führt wie eigene Versuche zeigen, nicht zum gewünschten Erfolg.

Überraschenderweise gelingt der selektive Schutz der 3β-Hydroxygruppe der Verbindungen der allgemeinen Formel I, wenn man diese Substanzen mit Trimethylacetanhydrid — vorzugsweise unter Verwendung von 4-Dimethylaminopyridin oder 4-Pyrrolidinopyridin als Katalysator — durchführt, oder diese Verbindungen mit tert.-Butyl-dimethylsilylchlorid, mit Dimethyl-2-(3-methyl-butyl)-silylchlorid oder Tribenzylsilylchlorid — vorzugsweise unter Verwendung organischer Basen (Pyridin, Imidazol, Triethylamin, 4-Dimethylaminopyridin, Lutidin, Collidin etc.) — durchführt.

Die nachfolgenden Beispiele dienen zur Erläuterung des erfindungsgemäßen Verfahrens und der gewerblichen Verwertbarkeit der Verfahrensprodukte zur Herstellung des bekannten aldosteron-antagonistisch wirksamen 6β,7β ; 15β,16β-Dimethylen-3-oxo-17α-pregn-4-en-21,17-carbolacton :

A) Ausführungsbeispiele betreffend die Herstellung der erfindungsgemäßen Verbindungen :

Beispiel 1

Ein 2 Liter-Erlenmeyerkolben, der 500 ml einer 30 Minuten bei 120 °C im Autoklaven sterilisierten Nährlösung aus 1 % Stärkezucker und 1 % Sojabohnenmehl, eingestellt auf pH 6,2 wird mit einer Schrägröhrchen-Kultur des Stammes Botryodiplodia malorum (CBS 13450) beimpft und 2 1/2 Tage auf einem Rotationsschüttler geschüttelt.

Mit dieser Anzuchtkultur wird ein 20 Liter-Vorfermenter beimpft, der mit 15 l eines 60 Minuten bei 121 °C und 1,1 atü sterilisierten Mediums der gleichen Zusammensetzung wie die Anzuchtkultur gefüllt ist. Unter Zugabe von Silicon SH als Antischaummittel wird bei 29 °C und 0,7 atü Druck unter Belüftung (15 l/Min.) und Rühren (220 U/Min.) 24 Stunden germiniert.

Danach werden 0,9 Liter dieser Kultur unter sterilen Bedingungen entnommen und damit ein 20 Liter-Hauptfermenter beimpft, der mit 14 l eines wie oben sterilisierten Nährmediums der gleichen Zusammensetzung wie die Vorfermenterkultur beschickt ist. Nach einer Anwachsphase von 12 Stunden unter Vorfermenterbedingungen wird eine pasteurisierte Suspension von 30 g feinstgemahlenem 3β-Hydroxy-

15β,16β-methylen-5-androsten-17-on in 2 250 ml 1 %iger wässriger Tween-80-Lösung hinzugegeben und weiter gerührt und belüftet. Der Verlauf der Fermentation wird durch Entnahme von Proben kontrolliert, die mittels Methylisobutylketon extrahiert und dünnschichtchromatographisch analysiert werden. Nach 48 Stunden Kontaktzeit ist die Umsetzung des Substrates beendet. Die Kulturbrühe wird durch Zentrifugation in einer Durchlaufzentrifuge vom Pilzmycel befreit, das klare Filtrat dreimal mit je 10 l Methylisobutylketon extrahiert und die Extrakte mit dem Extrakt des gleichfalls mittels Methylisobutylketon extrahierten Mycels vereinigt. Nach Konzentrierung der Lösung im Umlaufverdampfer wird anschließend bei 50 °C Badtemperatur im Vakuum im Rotationsverdampfer zur Trockne eingeengt. Den öligkristallinen Rückstand nimmt man in Isopropyläther auf und saugt über Papierfilter ab. Nach vierstündigem Trocknen bei 70 °C im Vakuumtrockenschrank erhält man 23,6 g 3β,7β-Dihydroxy-15β,16β-methylen-5-androsten-17-on vom Schmelzpunkt 199 bis 202 °C.

Beispiel 2

Unter den Bedingungen des Beispiels 1 werden 22,5 g 3β-Acetoxy-15β,16β-methylen-5-androsten-17-on mit Botryodiplodia malorum fermentiert und hierbei 16,3 g 3β,7β-Dihydroxy-15β,16β-methylen-5-androsten-17-on vom Schmelzpunkt 197-200 °C erhalten.

Beispiel 3

Unter den Bedingungen des Beispiels 1 werden 22,5 g 3β-Hydroxy-15β,16β-methylen-17α-pregn-5-en-21,17-carbolacton mit Botryodiplodia malorum incubiert und dabei 16,8 g 3β,7β-Dihydroxy-15β,16β-methylen-17α-pregn-5-en-21,17-carbolacton vom Schmelzpunkt 173-175 °C erhalten.

Beispiel 4

Eine Lösung von 195 g 3β,7β-Dihydroxy-15β,16β-methylen-5-androsten-17-on in 1 460 ml Pyridin wird mit 293 ml Pivalinsäureanhydrid und 31 g Dimethylaminopyridin versetzt. Nach einer Reaktionszeit von 72 Stunden bei Raumtemperatur wird das Rohprodukt mit 15 l Eiswasser gefällt, abgesaugt, gewaschen und bei 60 °C getrocknet. Nach dem Umkristallisieren aus Aceton-Dichlormethan erhält man 185,4 g 7β-Hydroxy-15β,16β-methylen-3β-pivaloyloxy-5-androsten-17-on. Schmelzpunkt 236 °C. $[\alpha]_D = -45°$ (Chloroform).

Beispiel 5

Eine Lösung von 120 g 3β,7β-Dihydroxy-15β,16β-methylen-5-androsten-17-on in 1 000 ml Dimethylformamid wird mit 31 g Imidazol versetzt und auf − 20 °C abgekühlt. Dazu tropft man innerhalb von 2 Stunden 63 g tert.-Butyl-dimethyl-chlorsilan in 450 ml Dimethylformamid und rührt eine Stunde nach. Das Reaktionsprodukt wird mit natriumchloridhaltigem Wasser gefällt, abfiltriert, mit Wasser gewaschen und in Methylenchlorid gelöst. Die Lösung wird über Natriumsulfat getrocknet und eingedampft. Nach Chromatographie an Kieselgel mit Hexan-Aceton erhält man 128,8 g 3β-(tert.-Butyl-dimethylsilyloxy)-7β-hydroxy-15β,16β-methylen-5-androsten-17-on.

Beispiel 6

Eine Lösung von 100 g 3β,7β-Dihydroxy-15β,16β-methylen-5-androsten-17-on in 2,5 l Tetrahydrofuran und 250 ml Pyridin wird auf − 20 °C abgekühlt und mit 88 ml Dimethyl-2-(3-methylbutyl)-silylchlorid innerhalb einer Stunde versetzt und 4 Stunden bei dieser Temperatur gerührt. Danach wird mit 50 ml Methanol versetzt und im Vakuum eingeengt. Das Rohprodukt wird an Kieselgel mit Methylenchlorid-Aceton chromatographiert. Man erhält 98,5 g 3β-[Dimethyl-2-(3-methyl-butyl)-siloxy]-7β-hydroxy-15β,16β-methylen-5-androsten-17-on als Öl. $[\alpha]_D = -36°$ (Chloroform).

Beispiel 7

Eine Lösung von 15,7 g 3β,7β-Dihydroxy-15β,16β-methylen-5-androsten-17-on in 750 ml Methylenchlorid wird mit 9 ml Triethylamin und 200 mg Dimethylaminopyridin versetzt und auf − 20 °C abgekühlt. Dazu tropft man innerhalb von 30 Minuten 19,4 g Tribenzylsilylchlorid in 10 ml Methylenchlorid. Man rührt 1 Stunde bei − 20 °C und 1 Stunde bei 0 °C, verdünnt mit Methylenchlorid und wäscht mit 1 N Salzsäure, gesättigter Natriumhydrogencarbonatlösung und Wasser. Nach Trocknung über Natriumsulfat engt man im Vakuum ein. Nach Chromatographie an Kieselgel mit Hexan-Aceton erhält man 27,5 g 3β-Tribenzylsilyloxy-7β-hydroxy-15β,16β-methylen-5-androsten-17-on.

Beispiel 8

16,0 g 3β,7β-Dihydroxy-15β,16β-methylen-17α-pregn-5-en-21,17-carbolacton werden in 130 ml

Dimethylformamid gelöst, die Lösung wird mit 4 g Imidazol versetzt und auf – 20 °C gekühlt. Innerhalb von 30 Minuten tropft man 8 g tert.-Butyldimethylchlorsilan in 60 ml Dimethylformamid hinzu und rührt 2 Stunden unter weiterer Kühlung. Die Reaktionslösung wird in Wasser eingegossen, das ausgefällte Produkt abfiltriert, gewaschen und getrocknet. Das Rohprodukt wird mit Aceton-Hexan an Kieselgel chromatographiert. Ausbeute 15,4 g 3β-(tert.-Butyl-dimethylsilyloxy)-7β-hydroxy-15β,16β-methylen-17α-pregn-5-en-21,17-carbolacton.

B) Ausführungsbeispiele zur gewerblichen Verwertbarkeit der erfindungsgemäßen Verbindungen.

Beispiel 1

a) Eine Suspension von 160 g 7β-Hydroxy-15β,16β-methylen-3β-pivaloyloxy-5-androsten-17-on in 1 600 ml Toluol wird bei 80 °C, nach Zugabe von 1,6 g Vanadium(IV)-oxid-acetylacetonat, innerhalb von 2 Stunden mit 160 ml 80 %igen tert.-Butylhydroperoxid in 475 ml Toluol versetzt. Nach dem Abkühlen wird die Lösung mit Wasser gewaschen, über Natriumsulfat getrocknet und im Vakuum eingedampft. Ausbeute 171 g 5,6β-Epoxy-7β-hydroxy-15β,16β-methylen-3β-pivaloyloxy-5β-androstan-17-on. Eine aus Aceton-Hexan umkristallisierte Probe schmilzt bei 220 °C. $[\alpha]_D = -12°$ (Chloroform).

b) Eine Lösung von 169 g 5,6β-Epoxy-7β-hydroxy-15β,16β-methylen-3β-pivaloyloxy-5β-androstan-17-on in einem Gemisch aus je 340 ml Dichlormethan, Tetrachlormethan und Pyridin wird mit 200 g Triphenylphosphin versetzt und 2 Stunden bei Raumtemperatur gerührt. Die Reaktionslösung wird mit Wasser gewaschen, über Natriumsulfat getrocknet und im Vakuum bis zur Trockne eingeengt. Der Rückstand wird mit 310 ml Ethanol aufgerührt und filtriert. Der Filterkuchen wird mit 175 ml Ethanol gewaschen und im Vakuum getrocknet. Es werden 139,2 g 7α-Chlor-5,6β-epoxy-15β,16β-methylen-3β-pivaloyloxy-5β-androstan-17-on erhalten. Eine analytische Probe, aus Aceton-Hexan umkristallisiert, besitzt einen Schmelzpunkt von 228 °C. $[\alpha]_D = -100°$ (Chloroform).

c) Eine Lösung von 196 g 7α-Chlor-5,6β-epoxy-15β,16β-methylen-3β-pivaloyloxy-5β-androstan-17-on in 500 ml Essigsäure und 800 ml Tetrahydrofuran wird bei 70 °C mit 392 g Zinkstaub in 2 Portionen im Abstand von 30 Minuten versetzt und 1 Stunde bei dieser Temperatur gerührt. Nach dem Abkühlen wird das Zink über Celite abfiltriert und mit 5 l Methylenchlorid gewaschen. Die vereinigten Filtrate versetzt man mit 1,5 l Wasser und neutralisiert unter Rühren durch Zugabe von festem Natriumhydrogencarbonat. Anschließend wird die organische Phase mit Wasser gewaschen, über Natriumsulfat getrocknet und im Vakuum eingeengt. Durch Verreiben des erhaltenen Feststoffes mit Essigester erhält man 134,7 g 5-Hydroxy-15β,16β-methylen-3β-pivaloyloxy-5β-androst-6-en-17-on vom Schmelzpunkt 243 °C.

d) 134 g 5-Hydroxy-15β,16β-methylen-3β-pivaloyloxy-5β-androst-6-en-17-on werden in 1 340 ml Tetrahydrofuran und 670 ml Methanol gelöst und nacheinander mit 40 g pulverisierten Kaliumhydroxid und 13 g Natriumperchlorat versetzt. Nach 2,5 Stunden wird in 8 l Wasser eingerührt, mit 20 %iger Schwefelsäure neutralisiert und der ausgefallene Feststoff abfiltriert. Nach Lösen in Methylenchlorid und Trocknung mit Natriumsulfat engtman im Vakuum ein. Durch Verreiben des erhaltenen Feststoffes mit Essigester erhält man 99,8 g 3β,5-Dihydroxy-15β,16β-methylen-5β-androst-6-en-17-on vom Schmelzpunkt 198 °C.

e) Eine Lösung von 26 g 3β,5-Dihydroxy-15β,16β-methylen-5β-androst-6-en-17-on in 520 ml Ethylenglykoldimethylether wird mit 78 g Zink-Kupfer und 69 ml Methylenjodid 4 Stunden bei 80 °C gerührt. Anschließend wird mit Methylenchlorid verdünnt, mit gesättigter Ammoniumchloridlösung und Wasser gewaschen, getrocknet und eingedampft. Der Rückstand wird an Kieselgel chromatographiert. Es werden 16,3 g 3β,5-Dihydroxy-6β,7β ; 15β,16β-dimethylen-5β-androstan-17-on vom Schmelzpunkt 205,5 — 207 °C erhalten.

f) 25,1 g 3β,5-Dihydroxy-6β,7β ; 15β,16β-dimethylen-5β-androstan-17-on werden in 500 ml Tetrahydrofuran gelöst. Unter Kühlung auf 0 °C und unter Argonatmosphäre werden zu dieser Lösung 75,5 g Kaliummethylat gegeben und anschließend unter Rühren 50,4 ml Propargylalkohol in 104 ml Tetrahydrofuran gelöst zugetropft. Das Reaktionsgemisch wird 20 Stunden bei 0 °C gerührt und in Eiswasser eingegossen. Nach Neutralisation mit verdünnter Schwefelsäure wird der ausgefallene Niederschlag abfiltriert und getrocknet. Das Rohprodukt wird an Kieselgel chromatographiert. Man erhält 25 g 17α-(3-Hydroxy-1-propinyl)-6β,7β ; 15β,16β-dimethylen-5β-androstan-3β,5,17β-triol. Schmelzpunkt 202-203 °C (Aceton).

g) 24,5 g 17α-(3-Hydroxy-1-propinyl)-6β,7β ; 15β,16β-dimethylen-5β-androstan-3β,5,17β-triol werden in 250 ml Tetrahydrofuran und 125 ml Methanol in Gegenwart von 3,75 g Palladium auf Kohle (10 %ig) und 0,5 ml Pyridin bis zur Aufnahme von 2 Äquivalenten Wasserstoff hydriert. Es wird vom Katalysator abfiltriert und eingedampft. Man erhält 24,7 g 17α-(3-Hydroxypropyl)-6β,7β ; 15β,16β-dimethylen-5β-androstan-3β,5,17β-triol, das ohne weitere Aufreinigung in die nächste Stufe eingesetzt wird.

h) Eine Lösung von 24,7 g 17α-(3-Hydroxypropyl)-6β,7β ; 15β,16β-dimethylen-5β-androstan-3β,5,17β-triol in 247 ml Pyridin wird mit einer Lösung von 74,1 g Chrom(VI)-oxid in 247 ml Wasser und 494 ml Pyridin versetzt und 16 Stunden bei 50 °C gerührt. Danach wird mit Methylenchlorid verdünnt, mit Wasser gewaschen, getrocknet und eingedampft. Der Rückstand wird an Kieselgel chromatographiert. Nach Umkristallisieren aus Di-isopropylether-Aceton werden 14,5 g 6β,7β ; 15β,16β-Dimethylen-3-oxo-17α-pregn-4-en-21,17-carbolacton vom Schmelzpunkt 196,5 bis 197,5 °C erhalten.

Beispiel 2

a) 128 g 3β-(tert.-Butyl-dimethylsilyloxy)-7β-hydroxy-15β,16β-methylen-5-androsten-17-on werden in 750 ml Toluol gelöst und bei 80 °C mit 500 mg Vanadin(IV)-oxid-acetylacetonat versetzt. Dazu tropft man 460 ml tert.-Butyl-hydroperoxidlösung (aus 100 ml 80 %igem tert.-Butyl-hydroperoxid und 400 ml Toluol) und beläßt für 1,5 Stunden bei dieser Temperatur. Nach dem Abkühlen wäscht man mit Wasser und gesättigter Natriumchloridlösung, trocknet über Natriumsulfat und dampft im Vakuum ein. Man erhält 126,5 g 3β-(tert.-Butyl-dimethylsilyloxy)-5,6β-epoxy-7β-hydroxy-15β,16β-methylen-5β-androstan-17-on vom Schmelzpunkt 189 °C. $[\alpha]_D = -8{,}8°$ (Chloroform).

b) 126 g 3β-(tert.-Butyl-dimethylsilyloxy)-5,6β-epoxy-7β-hydroxy-15β,16β-methylen-5β-androstan-17-on werden in einer Mischung von 600 ml Methylenchlorid, 600 ml Tetrachlorkohlenstoff und 300 ml Pyridin gelöst und mit 193,8 g Triphenylphosphin 2,5 Stunden gerührt. Anschließend wäscht man mit Wasser, trocknet über Natriumsulfat und engt im Vakuum ein. Nach Chromatographie an Kieselgel mit Hexan-Aceton erhält man 117,4 g 3β-(tert.-Butyl-dimethylsilyloxy)-7α-chlor-5,6β-epoxy-15β,16β-methylen-5β-androstan-17-on vom Schmelzpunkt 155,5 °C. $[\alpha]_D = -87°$ (Chloroform).

c) 142 g 3β-(tert.-Butyl-dimethylsilyloxy)-7α-chlor-5,6β-epoxy-15β,16β-methylen-5β-androstan-17-on werden in 400 ml Tetrahydrofuran und 400 ml Methanol gelöst und mit 1 000 ml 8 %iger Schwefelsäure 1,5 Stunden bei Raumtemperatur gerührt. Anschließend verdünnt man mit Ether, wäscht mit gesättigter Natriumhydrogencarbonatlösung und Wasser, trocknet über Natriumsulfat und engt im Vakuum ein. Durch Verreiben des erhaltenen Feststoffes mit Diisopropylether erhält man 108 g 7α-Chlor-5,6β-epoxy-3β-hydroxy-15β,16β-methylen-5β-androstan-17-on vom Schmelzpunkt 173 °C.

d) 107,5 g 7α-Chlor-5,6β-epoxy-3β-hydroxy-15β,16β-methylen-5β-androstan-17-on wird in 500 ml Essigsäure und 500 ml Tetrahydrofuran gelöst und nach Zugabe von 324 g Zinkstaub in 3 Portionen bei Raumtemperatur 16 Stunden gerührt. Anschließend wird das Zink abfiltriert, das Filtrat mit Methylenchlorid verdünnt und mit Natriumhydrogencarbonatlösung und Wasser gewaschen. Nach Trocknung über Natriumsulfat engt man im Vakuum ein. Durch Verreiben des erhaltenen Feststoffes mit Essigester erhält man 91,5 g 3β,5-Dihydroxy-15β,16β-methylen-5β-androst-6-en-17-on vom Schmelzpunkt 196 °C.

Beispiel 3

a) Eine Lösung von 133 g 3β-[Dimethyl-2-(3-methyl-butyl)-silyoxy]-7β-hydroxy-15β,16β-methylen-5-androsten-17-on in 740 ml wird bei 80 °C mit 624 mg Vanadin(IV)-oxid-acetylacetonat versetzt. Dazu tropft man 470 ml tert.-Butylhydroperoxidlösung (aus 94 ml 80 %igen tert.-Butylhydroperoxid und 390 ml Toluol) innerhalb von 2 Stunden. Die Lösung wird eine Stunde bei dieser Temperatur nachgerührt. Nach dem Abkühlen wäscht man mit Wasser und gesättigter Natriumchloridlösung, trocknet über Natriumsulfat und dampft im Vakuum ein. Man erhält 135 g 3β-[Dimethyl-2-(3-methyl-butyl)-silyloxy]-5,6β-epoxy-7β-hydroxy-15β,16β-methylen-5β-androstan-17-on als Öl. $[\alpha]_D = -16°$ (Chloroform).

b) 134 g 3β-[Dimethyl-2-(3-methyl-butyl)-silyloxy]-5,6β-epoxy-7β-hydroxy-15β,16β-methylen-5β-androstan-17-on werden in einer Mischung von 1 200 ml Methylenchlorid, 1 200 ml Tetrachlorkohlenstoff und 600 ml Pyridin gelöst. Die Lösung wird mit 140 g Triphenylphosphin versetzt und 3 Stunden bei Raumtemperatur gerührt. Anschließend wäscht man mit Wasser, trocknet über Natriumsulfat und engt im Vakuum ein. Nach Chromatographie an Kieselgel mit Hexan-Aceton erhält man 126,4 g 7α-Chlor-3β-[dimethyl-2-(3-methyl-butyl)-silyloxy]-5,6β-epoxy-15β,16β-methylen-5β-androstan-17-on als Öl. $[\alpha]_D = -72°$ (Chloroform).

c) Eine Lösung von 107 g 7α-Chlor-3β-[dimethyl-2-(3-methylbutyl)-silyloxy]-5,6β-epoxy-15β,16β-methylen-5β-androstan-17-on in einer Mischung von 500 ml Tetrahydrofuran und 500 ml Methanol wird mit 100 ml 8 %iger Schwefelsäure versetzt und 1,5 Stunden bei Raumtemperatur gerührt. Anschließend verdünnt man mit Ether, wäscht mit gesättigter Natriumhydrogencarbonatlösung und Wasser, trocknet über Natriumsulfat und engt im Vakuum ein. Durch Verreiben des erhaltenen Feststoffes mit Diisopropylether erhält man 74,5 g 7α-Chlor-5,6β-epoxy-3β-hydroxy-15β,16β-methylen-5β-androstan-17-on vom Schmelzpunkt 173,5 °C.

Beispiel 4

a) 24 g 3β-Tribenzylsilyloxy-7β-hydroxy-15β,16β-methylen-5-androsten-17-on werden in 50 ml Toluol gelöst, mit 500 mg Vanadin(IV)-oxid-acetylacetonal versetzt und auf 80 °C erhitzt. Dazu tropft man 75 ml tert.-Butylhydroperoxidlösung (10 ml 80 %igen tert.-Butylhydroperoxid in 100 ml Toluol) und beläßt für 1,5 Stunden bei dieser Temperatur. Nach dem Abkühlen wäscht man mit Wasser und gesättigter Natriumchloridlösung, trocknet und dampft im Vakuum ein. Man erhält 24,5 g 3β-Tribenzylsilyloxy-5,6β-epoxy-7β-hydroxy-15β,16β-methylen-5β-androstan-17-on.

b) 24 g 3β-Tribenzylsilyloxy-5,6β-epoxy-7β-hydroxy-15β,16β-methylen-5β-androstan-17-on werden in einem Gemisch von 250 ml Methylenchlorid, 250 ml Tetrachlorkohlenstoff und 125 ml Pyridin gelöst und mit 24 g Triphenylphosphin 3 Stunden gerührt. Anschließend wäscht man mit Wasser, trocknet über Natriumsulfat und engt im Vakuum ein. Nach Chromatographie an Kieselgel mit Hexan-Aceton erhält man

19,8 g 3β-Tribenzylsilyloxy-7α-chlor-5,6β-epoxy-15β,16β-methylen-5β-androstan-17-on.

c) 19 g 3β-Tribenzylsilyloxy-7α-chlor-5,6β-epoxy-15β,16β-methylen-5β-androstan-17-on werden in 50 ml Tetrahydrofuran und 50 ml Methanol gelöst und mit 15 ml 8 %iger Schwefelsäure 2 Stunden bei Raumtemperatur gerührt. Anschließend verdünnt man mit Ether, wäscht mit gesättigter Natriumhydrogencarbonatlösung und Wasser, trocknet über Natriumsulfat und engt im Vakuum ein. Durch Verreiben des erhaltenen Feststoffes mit Diisopropylether erhält man 8,7 g 7α-Chlor-5,6β-epoxy-3β-hydroxy-15β, 16β-methylen-5β-androstan-17-on vom Schmelzpunkt 172 °C.

Beispiel 5

a) 15,0 g 3β-(tert.-Butyl-dimethylsilyloxy)-7β-hydroxy-15β,16β-methylen-17α-pregn-5-en-21,17-carbolacton werden analog den Vorschriften 2 a) bis 2 d) in 3β, 5-Dihydroxy-15β, 16β-methylen-5β, 17α-pregn-6-en-21,17-carbolacton überführt. Ausbeute 10,3 g. Schmelzpunkt 236 °C.

b) 5 g 3β,5-Dihydroxy-15β,16β-methylen-5β,17α-pregn-6-en-21,17-carbolacton werden in 100 ml Tetrahydrofuran gelöst und mit 15 g Zink-Kupfer versetzt. Dazu tropft man innerhalb von 7 Stunden 13,2 ml Methylenjodid so zu, daß die Temperatur 30 °C nicht übersteigt und rührt weitere 10 Stunden bei Raumtemperatur. Zur Entfernung des Metalls wird über Celite filtriert, das Filtrat mit Methylenchlorid verdünnt und mit gesättigter Ammoniumchloridlösung gewaschen, über Magnesiumsulfat getrocknet und im Vakuum eingeengt. Der Rückstand wird an Kieselgel chromatographiert. Man erhält 4,4 g 3β,5-Dihydroxy-6β,7β ; 15β,16β-dimethylen-5β,17α-pregnan-21,17-carbolacton als Öl.

c) 2,8 g 3β,5-Dihydroxy-6β,7β ; 15β,16β-dimethylen-5β,17α-pregnan-21,17-carbolacton werden in 28 ml Pyridin gelöst und mit einer Lösung von 15 g Chrom(VI)-oxid in 28 ml Pyridin und 14 ml Wasser versetzt und 16 Stunden bei 50 °C gerührt. Nach dem Abkühlen wird mit Methylenchlorid verdünnt, mit Wasser gewaschen, über Magnesiumsulfat getrocknet und im Vakuum eingeengt. Der Rückstand wird an Kieselgel chromatographiert. Man erhält 2,3 g 6β,7β ; 15β,16β-Dimethylen-3-oxo-17α-pregn-4-en-21,17-carbolacton vom Schmelzpunkt 198 bis 198,5 °C.

**Ansprüche**

1. Verfahren zur Herstellung von 3β,7β-Dihydroxy-$\Delta^5$-steroiden der allgemeinen Formel I

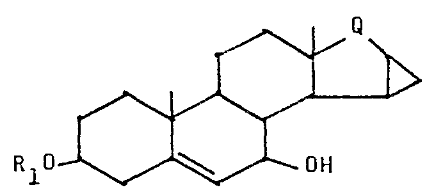

(I)

worin Q die Gruppierungen

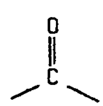 , oder 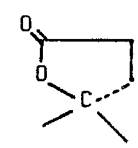

und $R_1$ ein Wasserstoffatom, eine Trimethylacetylgruppe, eine tert.-Butyl-dimethylsilylgruppe, eine Dimethyl-2-(3-methylbutyl)-silylgruppe oder eine Tribenzylsilylgruppe darstellen, dadurch gekennzeichnet, daß man ein 3β-Hydroxy-$\Delta^5$-steroid der allgemeinen Formel II

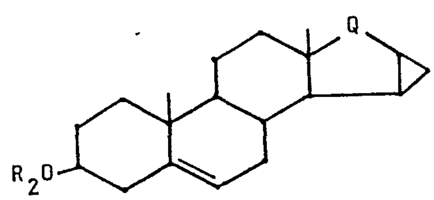

(II)

worin Q die obengenannte Bedeutung besitzt und $R_2$ ein Wasserstoffatom oder eine Alkanoylgruppe mit 2 bis 6 Kohlenstoffatomen darstellt, mit einer Kultur von Botryodiplodia malorum, welche die gewünschte Hydroxylierung durchzuführen vermag, fermentiert, und gewünschtenfalls die erhaltenen 3β,7β-Dihydroxy-$\Delta^5$-steroide der allgemeinen Formel I mit Trimethylacetanhydrid, tert.-Butyl-dimethylsilylchlorid,

Dimethyl-2-(3-methyl-butyl)-silylchlorid oder Tribenzylsilylchlorid umsetzt.

2. Verfahren zur Herstellung von 3β,7β-Dihydroxysteroiden gemäß Anspruch 1, dadurch gekennzeichnet, daß man die Fermentation mit einer Kultur von Botryodiplodia malorum (CBS 13450) durchführt.

**Claims**

1. Process for the manufacture of 3β,7β-dihydroxy-$\Delta^5$-steroids of the general formula I

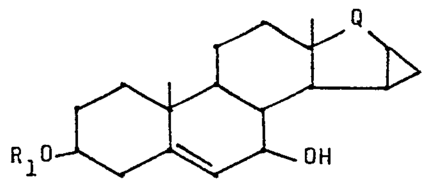

(I)

in which Q represents the groupings

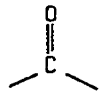

, or

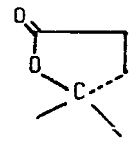

and $R_1$ represents a hydrogen atom, a trimethylacetyl group, a tert.-butyl-dimethyl-silyl group, a dimethyl-2-(3-methylbutyl)-silyl group or a tribenzylsilyl group, characterised in that a 3β-hydroxy-$\Delta^5$-steroid of the general formula II

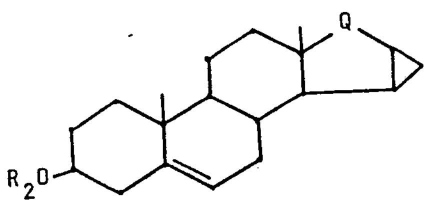

(II)

in which Q has the meaning given above and $R_2$ represents a hydrogen atom or an alkanoyl group having from 2 to 6 carbon atoms, is fermented with a culture of Botryodiplodia malorum, which is capable of effecting the desired hydroxylation, and, if desired, the resulting 3β,7β-dihydroxy-$\Delta^5$-steroids of the general formula I are reacted with trimethylacetic anhydride, tert.-butyl-dimethyl-silyl chloride, dimethyl-2-(3-methylbutyl)-silyl chloride or tribenzylsilyl chloride.

2. Process for the manufacture of 3β,7β-dihydroxy steroids according to claim 1, characterised in that the fermentation is carried out using a culture of Botryodiplodia malorum (CBS 13450).

**Revendications**

1. Procédé de préparation de dihydroxy-3β,7β-$\Delta^5$-stéroïdes répondant à la formule générale (I)

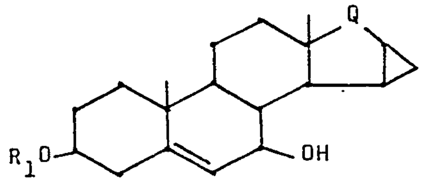

(I)

dans laquelle Q représente l'un des groupements

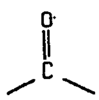

, et

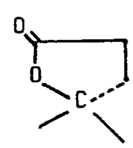

et $R_1$ représente un atome d'hydrogène ou un radical triméthylacétyle, tert-butyl-diméthylsilyle, diméthyl-(diméthyl-1,2 propyl)-silyle ou tribenzylsilyle, procédé caractérisé en ce qu'on fait fermenter un hydroxy-3β $\Delta^5$-stéroïde répondant à la formule générale (II)

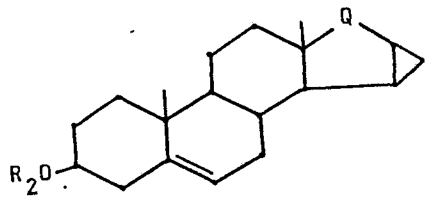

(II)

dans laquelle Q a la signification indiquée ci-dessus et $R_2$ représente un atome d'hydrogène ou un radical alcanoyle contenant de 2 à 6 atomes de carbone, avec une culture de Botryodiplodia malorum capable d'effectuer l'hydroxylation voulue, et, si on le désire, on fait réagir les dihydroxy-3β,7β-$\Delta^5$-stéroïdes de formule générale (I) ainsi obtenus avec l'anhydride triméthylacétique, le chlorure de tert-butyl-diméthylsilyle, le chlorure de diméthyl-(diméthyl-1,2 propyl)-silyle ou le chlorure de tribenzylsilyle.

2. Procédé de préparation de dihydroxy-3β,7β stéroïdes selon la revendication 1, procédé caractérisé en ce qu'on effectue la fermentation au moyen d'une culture de Botryodiplodia malorum CBS 13450.